# EUROPEAN PATENT SPECIFICATION

(11) **EP 2 718 364 B1**
(45) Date of publication and mention of the grant of the patent: **02.11.2016**
(21) Application number: 12725282.3
(22) Date of filing: 30.05.2012
(51) Int. Cl.: C08L 23/08, C10M 145/08

(54) **METHOD T0 MAKE AN AQUEOUS POUR POINT DEPRESSANT DISPERSION COMPOSITION**
VERFAHREN ZUR HERSTELLUNG EINER WÄSSRIGEN STOCKPUNKTERNIEDRIGENDEN DISPERSIONSZUSAMMENSETZUNG
PROCÉDÉ DE FABRICATION D'UNE COMPOSITION AQUEUSE DE DISPERSION AMÉLIORANT LE POINT D'ÉCOULEMENT

(30) Priority: 10.06.2011 US 201161495529 P
(43) Date of publication of application: 16.04.2014
(73) Proprietor: Dow Global Technologies LLC, Midland, MI 48674 (US)
(72) Inventor: STRANDBURG, Gary M., Mount Pleasant, MI 48858 (US); BLOXOM, Stephanie A., Beaverton, MI 48612 (US); KUHLMAN, Roger L., Lake Jackson, TX 77566 (US); MICHALOWSKI, Jeffrey D., Midland, MI 48642 (US); MIELCAREK, Melissa A., Saginaw, MI 48604 (US); SCHMIDT, Dale C., Midland, MI 48642 (US)
(74) Representative: Beck Greener
(86) International application number: PCT/US2012/039910
(87) International publication number: WO 2012/170242

(56) References cited:
- EP-A2- 0 173 456
- WO-A2-2009/045731
- US-A1- 2007 292 705

## Description

### FIELD OF THE INVENTION

The present invention relates to a pour point depressant composition comprising a thermoplastic polymer, in an aqueous medium which provides a lower pour point in crude oils, exists as a liquid over a broad temperature range, and demonstrate long term stability to creaming, and a method for the preparation of said pour point depressant composition.

### BACKGROUND OF THE INVENTION

Various types of crude oils require the use of pour point depressant additives in order to improve flow at low temperatures. Many pour point materials are waxy solids at ambient production site temperatures. To be able to pump these waxy materials down hole, they need to be converted into a liquid form, for example by dissolving them or dispersing them into a liquid medium. Kerosene has been used as such a medium. However, kerosene is not desirable for a variety of reasons, including flammability, environmental impact, and economic considerations.

When used in cold climates, these solutions or dispersions of pour point depressants must also remain as liquids at low temperatures, such as -10°C, or lower. One solution has been to disperse the waxy pour point depressants in a mixture of water and a glycol such as ethylene glycol or propylene glycol. Using water is economical and it also adds to safety by not requiring the use of a flammable liquid medium.

In addition to other classes of polymers, copolymers of ethylene and vinylesters of C₁ to C₄ fatty acids, usually comprising a vinyl ester content of 15 percent to 40 percent by weight, have shown to be effective flow improvers for crude oils and middle distillates (these are the so-called ethylene vinyl acetate copolymers (EVA-copolymers), for example see USP 3,048,479; 3,567,639; and 3,669,189. For cold climate conditions, it is desirable that an aqueous EVA dispersion have a high solids content (e.g., greater than 25 percent EVA) while still being able to flow at low temperatures (-10°C or below).

However, dispersing EVA into an aqueous dispersion containing ethylene glycol is problematic. For some EVA polymers, especially those containing a low fraction of vinyl acetate, the polymer has a high melting point and without the use of a solvent, the EVA is not adequately dispersed by conventional dispersion techniques. Furthermore, EVA dispersions typically do not exhibit long term stability against creaming (migration toward the top of the carrier medium).

There is a need for a pour point depressant comprising suitable additives dispersed in a medium free of flammable solvents, which provides a lowering of the pour point in crude oils, exists as a liquid over a broad temperature range (ambient to 40°C), and demonstrates long term stability to creaming.

### SUMMARY OF THE INVENTION

The present invention is a method to make such as a pour point depressant compositon comprising the steps of (a) combining in an extruder: i) ethylene vinyl acetate copolymer (EVA), preferably in an amount of from 12 to 50 weight percent; ii) a dispersing agent, preferably a sodium salt of oleic acid, stearic acid, behenic acid or erucic acid or a potassium salt of oleic acid, stearic acid, behenic acid or erucic acid, or mixtures thereof, preferably in an amount of from 1 to 10 weight percent; iii) water to form an aqueous dispersion of EVA, optionally v) a stabilizing agent, preferably a polyvinyl alcohol; or an ionomer of an ethylene acrylic acid copolymer, an ethylene methacrylic acid copolymer, a polyacrylic acid polymer, a polyacrylic acid co-polymer, or an acrylic and urethane copolymer; and more preferably a polyethoxylated nonionic surfactant, preferably in an amount of from 0.5 weight percent to 5 weight percent; and optionally vi) an additional additive selected from a biocide, a colorant, an anti-foaming agent, or a mixture thereof, and optionally (b) mixing the aqueous dispersion of EVA with iv) an aqueous freezing point depressant, preferably sodium chloride, potassium chloride, calcium chloride, methanol, ethanol, propanol, propylene glycol, glycerine, ethyl ether of ethylene glycol, propyl ether of ethylene glycol, butyl ether of ethylene glycol, hexyl ether of ethylene glycol, diethylene glycol, or propylene glycol, and more preferably ethylene glycol; wherein weight percents are based on the total weight of the aqueous pour point depressant dispersion composition and preferably the volume average particle size of the dispersed EVA is equal to or less than 1 micrometers, the water: aqueous freezing point depressant mixture is present in an amount of from 40 to 75 weight percent, and the water:aqueous freezing point depressant ratio is from 40:60 to 70:30.

Preferably in the method to make an aqueous pour point depressant dispersion composition disclosed herein above, the dispersing agent ii) comprises one or more of: a) a fatty acid/salt having the formula R₁COOR₂ wherein R₁ is a straight chain, saturated or unsaturated, hydrocarbon radical of 8 to 25 carbon atoms and R₂ is H or a base-forming radical; b) an alkyl, arene and/or alkylarene sulfonate; c) a salt of a polymer of alkyl acrylate and/or alkyl methacrylate and acrylic and/or methacrylic acid, or a salt of partial esters of maleic anhydride-styrene copolymers; d) a cationic surfactant; e) a zwitterionic surfactant; or f) a nonionic surfactant.

Preferably in the method to make an aqueous pour point depressant dispersion composition disclosed herein above, steps a) and b) are conducted sequentially in the extruder in which the aqueous dispersion of EVA is produced.

Preferably in the method to make an aqueous pour point depressant dispersion composition disclosed herein above, step b) does not occur in the extruder in which the aqueous dispersion of EVA is produced.

Preferably in the method to make an aqueous pour point depressant dispersion composition comprising a salt disclosed herein above, the salt is formed in the extruder.

### BRIEF DESCRIPTION OF DRAWINGS

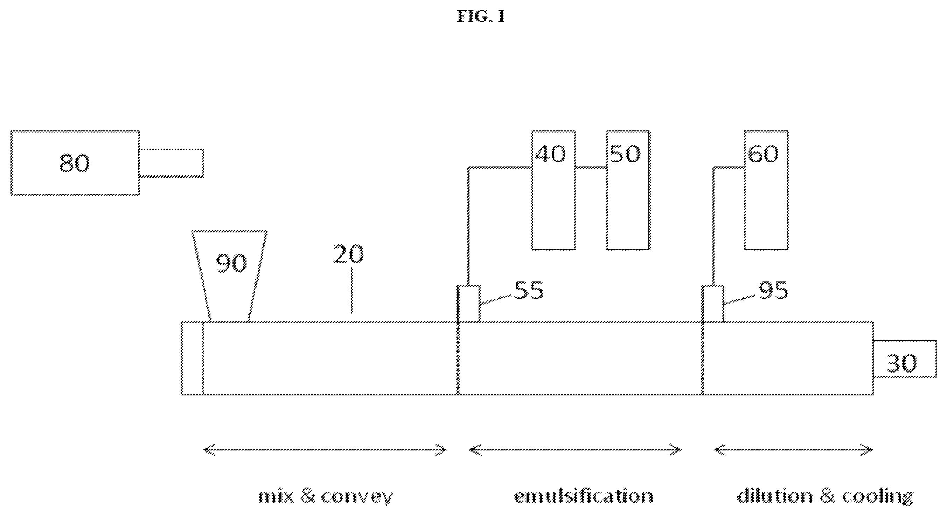

**FIG.1** is a schematic representation of a typical melt-extrusion apparatus used to prepare the aqueous pour point depressant dispersion compositons of the present invention.

### DETAILED DESCRIPTION OF THE INVENTION

Component i, useful as the the pour point depressant of the present invention, is a thermoplastic polymer. A suitable thermoplastic polymer for the present invention is a copolymer of ethylene with at least one vinyl ester of a saturated aliphatic C₁ to C₂₄ - carboxylic acid, for example, see USP 3,382,055. In such polymers, different vinyl esters can concurrently be used. The polymers can in principle be prepared by bulk, emulsion, or solution polymerization. As comonomers, for example, vinyl esters of acetic acid, propionic acid, butyric acid, 2-ethylhexane carboxylic acid, pelargonic acid, and stearic acid, particularly C₂ to C₄ -carboxylic acids, and especially vinyl acetate, can be used. A preferred thermoplastic polymer is an ethylene vinyl acetate copolymer. In general, the vinylester content is in the range from 10 to 80 percent, preferably 20 to 45 percent, more preferably 25 to 32 percent, more preferably 28 to 32 percent by weight.

Copolymers having a vinyl ester content less than 30 percent may be suitably prepared by a bulk high-pressure process.

Copolymers having from 3 to 20 molar parts of ethylene per molar part of vinyl acetate, having a molecular weight of 1,000 to 2,900, having a slight degree of branching of the ethylene chains, and prepared by free radical solution polymerization are described in German Patent Publication No. 1,914,756. The melt viscosity index, determined according to ASTM Test-Method D 1238-6 T, is between 1 and 800 grams per 10 minutes (g/10 min), preferably 5 to 400 g/10 min, more preferably 5 to 150 g/10 min. Commercially available ethylene vinyl acetate copolymers comprising 2 to 45 percent by weight of vinyl acetate and having a melt viscosity index of 6 to 150 g/10 min, such as are sold under the name ELVAX™ from DuPont, are useful in the present invention. Unless otherwise noted, melt viscosity index is determined according to ASTM D1238 at 190°C and a load of 2.16 kilograms (kg).

Said copolymers of ethylene with at least one vinyl ester of a saturated aliphatic carboxylic acid of the present invention are insoluble at room temperature in the carrier medium.

Preferably the thermoplastic polymer used in the aqueous pour point depressant disersion composition of the present invention is used in an amount equal to or greater than 20 weight percent, more preferably in an amount equal to or greater than 25 weight percent, and more preferably in an amount equal to or greater than 30 weight percent based on the total weight of the aqueous pour point depressant dispersion composition. Preferably the thermoplastic polymer used in the aqueous pour point depressant dispersion composition of the present invention is used in an amount equal to or less than 65 weight percent, more preferably in an amont equal to or less than 60 weight percent, and more preferably in an amount equal to or less than 55 weight percent based on the total weight of the aqueous pour point depressant dispersion composition.

In addition to the thermoplastic resin, dispersions described herein include a dispersing agent. As used here in the term "dispersing agent" means an agent that aids in the formation and/or stabilization of a dispersion. Some dispersing agents can also be used to form emulsions and are described in detail by Paul Becher (Emulsions: Theory and Practice, 3rd edition, Oxford University, New York, 2001), incorporated herein by reference in its entirety. Suitable dispersing agents, sometimes referred to as surfactants, for use in the present invention as component ii can be classified as anionic, cationic, zwitterionic, or non-ionic. Anionic surfactants include substances containing a long lipophilic tail bonded to a water-soluble (hydrophilic) group, wherein the hydrophilic group contains an anionic moiety such as a carboxylic acid, sulfonic acid, or phenolic group, neutralized by a cation such as an alkali metal or ammonium. The lipophilic tail is preferably an alkyl group, typically having about 8 to about 25 carbon atoms.

Typical anionic surfactants include carboxylic acids or salts thereof such as fatty acids/salts having the formula R₁COOR₂ wherein R₁ is a straight chain, saturated or unsaturated, hydrocarbon radical of about 8 to about 25 carbon atoms and R₂ is H or a base-forming radical such as Li, Na, K, or N R₄ (R is independently hydrogen, alkyl, aryl or arylalkyl). Alternatively R₂ may be a divalent or polyvalent metal, in which case the appropriate number of acid groups is normally present in order to provide the neutral salt. Multiply valent metal ions include Mg, Ca, Sr, Ba, Cr, Mn, Fe, Co, Ni, Cu, Zn, Sn, Pb, and others. Typical fatty acid salts include sodium stearate, sodium palmitate, ammonium oleate, and triethanolamine palmitate. Additional carboxylic acids/salts useful as anionic surfactants include acids/salts, and especially sodium and potassium salts, of coconut oil fatty acids and tall oil acids as well as other carboxylic acids salt compounds including amine salts such as triethanolamine salts, acylated polypeptides, and salts of N-lauryl sarcosine such as N-dodecanoyl-N-methylglycine sodium salt. Preferred dispersing agents in the present invention are behenic acid (R₁ = C₂₁H₄₃); erucic acid (R₁ = C₂₁H₄₁); sodium or potassium salts of oleic acid, stearic acid, behenic acid or erucic acid and/or mixtures thereof. Erucic acid may be for example in the form of rapeseed oil, a natural oil that contains approximately 40 to 50 weight percent erucic acid with the remainder consisiting primarily of chains having 18 carbon atoms.

Other anionic surfactants include alkyl, arene and alkylarene sulfonates such as alkylbenzene sulfonate, linear alkylbenzene sulfonates, sodium tetrapropylene benzene sulfonate, sodium dodecylbenzene sulfonate, benzene-, toluene-, xylene-, and cumene sulfonates, lignin sulfonates, petroleum sulfonates, paraffin sulfonates, secondary nalkanesulfonates, alpha-olefin sulfonates, alkylnaphthalene sulfonates; n-acyl-n-alkyltaurates; sulfosuccinate esters; isothionates; alkyl sulfates having the formula R₁OSO₃ R₂ wherein R₁ and R₂ are defined above, such as lithium dodecyl sulfate, sodium dodecyl sulfate, potassium dodecyl sulfate, and sodium tetradecyl sulfate; alkyl sulfonates having the formula R₁SO₃ R₂ wherein R₁ and R₂ are as defined above, such as sodium lauryl sulfonate; sulfated and sulfonated amides and amines; sulfated and sulfonated esters such as lauric monoglyceride sodium sulfate, sodium sulfoethyl oleate, and sodium lauryl sulfoacetate; sulfuric acid ester salts such as sulfated linear primary alcohols, sulfated polyethoxylated straight chain alcohols and sulfated triglyceride oils; phosphoric and polyphosphoric acid esters; perfluorinated carboxylic acids; and polymeric anionic surfactants such as alginic acids.

Also included are polymeric anionic surfactants such as salts of polymers of alkyl acrylates and/or alkyl methacrylates and acrylic and/or methacrylic acid, and salts of partial esters of maleic anhydride-styrene copolymers. An anionic surfactant may be the salt of an acid precursor reacted with a basic material to form the salt. Preferably, the acid precursor is neutralized in situ to form the salt.

Another group of materials which can be classified as anionic surfactants are those materials known as overbased or superbased materials. These are basic metal salts, preferably alkali or alkaline earth metal salts, of acidic organic compounds (carboxylic acids, sulfonic acids, phosphonic acids, phenols, and so on). Overbased materials are generally single phase, homogeneous Newtonian systems characterized by a metal content in excess of that which would be present for neutralization according to the stoichiometry of the metal and the particular acidic organic compound reacted with the metal. The overbased materials are prepared by reacting an acidic material (typically an inorganic acid or lower carboxylic acid, preferably carbon dioxide) with a mixture comprising an acidic organic compound, a reaction medium comprising at least one inert, organic solvent (mineral oil, naphtha, toluene, xylene, etc.) for said acidic organic material, a stoichiometric excess of a metal base, and a promoter such as a phenol or alcohol. The acidic organic material will normally have a sufficient number of carbon atoms to provide a degree of solubility in oil and to provide a measure of surfactant activity to the product. The amount of excess metal is commonly expressed in terms of metal ratio. The term "metal ratio" is the ratio of the total equivalents of the metal to the equivalents of the acidic organic compound: a neutral metal salt has a metal ratio of one; a salt having 4.5 times as much metal as present in a normal salt will have metal excess of 3.5 equivalents, or a ratio of 4.5.

Overbased materials are commonly used as lubricant additives and are well known to those skilled in the art. While they are useful for some applications, the scope of their utility may be different from that of other surfactants. That is, they have been observed occasionally to deposit what is believed to be calcium carbonate after exposure to an electric field. Nevertheless in situations where this is not a problem their use can be appropriate and they are accordingly considered to be within the scope of the present invention. Patents describing techniques for making basic salts of sulfonic acids, carboxylic acids, and mixtures of any two or more of these include USP 2,501,731; 2,616,905; 2,616,911; 2,616,925; 2,777,874; 3,256,186; 3,384,585; 3,365,396; 3,320,162; 3,318,809; 3,488,284; and 3,629,109, all of which are incorporated by reference herein in their entirety.

Cationic surfactants are similar to anionic surfactants except that the polar portion of the molecule has a positive charge. Examples of cationic surfactants include long-chain amines and their salts; such as primary amines derived from animal and vegetable fatty acids and tall oil and synthetic C₁₂ to C₁₈ primary, secondary, or tertiary amines; diamines and their salts, quaternary ammonium salts including tetraalkylammonium salts and imidazolinium salts derived from e.g. tallow or hydrogenated tallow, or N-benzyl-N-alkyl-dimethylammonium halides; polyethoxylated long-chain amines; quaternized polyethoxylated long-chain amines; and amine oxides such as N-alkyldimethylamine oxides (which are sometimes referred to as zwitterionic) such as cetyl dimethylamine oxide or stearyl dimethylamine oxide.

Zwitterionic surfactants include amino acids such as beta-N-alkylamino-propionic acids, N-alkyl-beta-iminodipropionic acids, imidazoline carboxylates, N-alkylbetaines, sulfobetaines, and sultaines.

Nonionic surfactants are materials in which the polar functionality is not provided by an anionic or cation group, but by a neutral polar group such as typically an alcohol, amine, ether, ester, ketone, or amide function. Typical nonionic surfactants include polyethoxylated alkylphenols such as polyethoxylated p-nonylphenol, p-octylphenol, or p-dodecylphenol; polyethoxylated straight-chain alcohols derived from coconut oil, tallow, or synthetic materials including oleyl derivatives; polyethoxylated polyoxypropylene glycols (block copolymers of ethylene oxide and propylene oxide), typically having molecular weights of 1000 to 30,000; polyethylene glycol; polyethoxylated mercaptans; long-chain carboxylic acid esters including glyceryl and polyglyceryl esters of natural fatty acids, propylene glycol esters, sorbitol esters, polyethoxylated sorbitol esters, polyoxyethylene glycol esters, and polyethoxylated fatty acids; alkanolamine "condensates" e.g. the condensates made by reaction of methyl or triglyceride esters of fatty acids with equimolar or twice equimolar amounts of alkanolamine; tertiary acetylenic glycols; polyethoxylated silicones, prepared by reaction of a reactive silicone intermediate with a capped allyl polyalkylene oxide such as propylene oxide or mixed ethylene oxide/propylene oxide copolymer; N-alkylpyrrolidones, and alkylpolyglycosides (long chain acetals of polysaccharides). Many of these and other ionic and non-ionic surfactants are discussed in Rosen, "Surfactants and Interfacial Phenomena," John Wiley & Sons, pp. 7-31, 1989.

Further nonionic surfactants more specifically include ethoxylated coco amide; oleic acid; t-dodecyl mercaptan; modified polyester dispersants; ester, amide, or mixed ester-amide dispersants based on polyisobutenyl succinic anhydride; dispersants based on polyisobutyl phenol; ABA type block copolymer nonionic dispersants; acrylic graft copolymers; octylphenoxypolyethoxyethanol; nonylphenoxypolyethoxyethanol; alkyl aryl ethers; alkyl aryl polyethers; amine polyglycol condensates; modified polyethoxy adducts; modified terminated alkyl aryl ethers; modified polyethoxylated straight chain alcohols; terminated ethoxylates of linear primary alcohols; high molecular weight tertiary amines such as 1-hydroxyethyl-2-alkyl imidazolines; oxazolines; perfluoralkyl sulfonates; sorbitan fatty acid esters; polyethylene glycol esters; aliphatic and aromatic phosphate esters. Also included are the reaction products of hydrocarbyl-substituted succinic acylating agents and amines. These reaction products and methods for preparing them are described in USP 4,234,435; 4,952,328; 4,938,881; and 4,957,649, all of which are incorporated herein by reference in their entirety.

Other nonionic surfactants include functionalized polysiloxanes. These materials contain functional groups such as amino, amido, imino, sulfonyl, sulfoxyl, cyano, hydroxy, hydrocarbyloxy, mercapto, carbonyl (including aldehydes and ketones), carboxy, epoxy, acetoxy, phosphate, phosphonyl, and haloalkyl groups. These polysiloxanes can be linear or branched and generally have molecular weight above 800, i.e. up to 10,000 or 20,000. The functionality can be randomly distributed on the polymer chain or present in blocks. The functionality can be present as alkyl or alkylaryl groups as well as groups such as-(C₂H₄O)ₐ --(C₃H₆O)_{b} --R where a and b are independently numbers from 0 to about 100 provided that at least one of a or b is at least 1, and R is H, acetoxy, or a hydrocarbyl group. Other suitable substituent groups can include C₃H₆X, where X is OH, SH, or NH₂. Examples of such materials include SILWET™ surfactants from Union Carbide and TEGOPREN™ silicone surfactants from Goldschmidt Chemical Corp., Hopewell, Va.

Nonionic surfactants include polyoxyalkenealkyl alcohols or phenols, such as ethoxylated nonylphenol; alkanoates (preferably partial alkanoates) of polyalcohols, such as glyceryl monooleate, glyceryl monolaurate, sorbitan monooleate, sorbitan sesquioleate, sorbitan monolaurate, and sorbitan sesquilaurate, and 4,4-bishydroxylmethyl-2-heptadecenyl-2-oxazoline. Preferred materials include tall oil fatty acid neutralized with diethanolamine; TRITON™ surface active agents (from The Dow Chemical Company), including the octylphenol series with 1 to 70 ethylene oxide units and the nonylphenol series with 4 to 40 ethylene oxide units; IGEPAL™ surfactants (from Rhone-Poulenc) containing 7 to 50 ethylene oxide units; TERGTITOL™ surfactants (from The Dow Chemical Company) containing 4 to 41 ethylene oxide units; and NEODOL ™ (from Shell Chemical Company) containing 3 to 13 ethylene oxide units. The foregoing commercial materials are generally linear primary alcohol ethoxylates, secondary alcohol ethoxylates, or (in the case of the TRITON materials) branched alkylphenol ethoxylates.

Preferably the dispersing agent used in the aqueous pour point depressant dispersion composition of the present invention is used in an amount equal to or greater than 1 weight percent, more preferably in an amount equal to or greater than 2 weight percent, and more preferably in an amount equal to or greater than 3 weight percent based on the total weight of the aqueous pour point depressant dispersion composition. Preferably the dispersing agent used in the aqueous pour point depressant dispersion composition of the present invention is used in amount equal to or less than 10 weight percent, more preferably in an amont equal to or less than 9 weight percent, and more preferably in an amount equal to or less than 8 weight percent based on the total weight of the aqueous pour point depressant dispersion composition.

The aqueous pour point depressant dispersion composition of the present invention optionally contains a stabilizing agent whose function is to maintain product stability across a broad spectrum of conditions, such as phase and particle stability at storage and shipping temperatures. Preferably, the dispersion is stable between 40°C and -40°C. The stabilizing agent may also provide shear stability protection to allow the product to be transferred via a number of different pumping systems. Suitable stabilizing agents may be monomeric surfactants, polymeric stabilizing agents, and/or mixtures thereof. Suitable monomer surfactants are disclosed herein above.

Preferred monomeric stabilizers are polyethoxylated nonionic surfactants. Most preferred are those having hydrophilic lipophillic balance (HLB) values of equal to or less than 16, more preferably HLB values equal to or less than 12, and most preferably those having HLB values equal to or less than 10. Not to be bound by theory, it is expected that the lower HLB nonionic surfactants adsorb to the particle of the dispersion better in the presence of the aqueous freezing point depressant.

HLB values are empirical numbers that indicate the emulsification properties of nonionic surfactants. An HLB value expresses the relative effect of the hydrophilic (water loving) portion of the surfactant (e.g., ethylene oxide chains) to the non-polar lipophilic (oil loving) portion. HLB values are generally based on experimental emulsification data. However they can be calculated in a variety of way, for example see "Surfactants and Interfacial Phenomena"; M.J. Rosen; Second Edition; John Wiley and Sons; 1989. For nonionic surfactants having just ethylene oxide chains as the hydrophilic portion, the HLB value is simply estimated by dividing the weight percent ethylene oxide by five.

A preferred nonionic surfactant for use in the present invention as a stabilizing agent is a molecule comprising two parts: a hydrophobic part or hydrophobe comprising hydrocarbyl groups and a hydrophilic part or hydrophile containing ethoxy (CH₂CH₂O) groups. The preferred surfactant for this invention has a hydrophobe that is either free of a phenolic group and contains 6 to 12 (more preferably 8 to 11) carbon atoms or that contains a phenolic group that is connected to 8 or 9 carbon atoms (also called an octyl phenol or a nonyl phenol, respectively) and the preferred surfactant has a hydrophile that contains 1 to 6 ethoxy groups (more preferably 2 to 4). Examples of these molecules include NEODOL™ surfactant ethoxylates (from Shell Chemical Co.) with 2 to 13 ethylene oxide units, for example an ethoxylated alcohol with a hydrophobe containing 9 to 11 carbon atoms and hydrophile, containing an average of 2.5 ethoxy groups (sold as NEODOL 91-2.5 by Shell), an ethoxylated alcohol with the hydrophobe containing a 2-ethylhexyl group and the hydrophobe containing an average of 3 ethoxy groups (sold as ECOSURF™ EH-3 by The Dow Chemical Company), and an ethoxylated nonyl phenol with 4 ethoxy groups (sold as TERGITOL™ NP-4 by The Dow Chemical Company). Preferably if an ethoxylated noinionic surfactant is used in the present invention, it is used in combination with one or more above disclosed dispersing agents.

Other polymeric stabilizers include polyvinyl alcohol or ionomers and/or salts of ethylene acrylic acid copolymers, ethylene methacrylic acid copolymers, polyacrylic acid polymers and co-polymers and associatitive types of acrylic and urethane co-polymers. The preferred polymeric stabilizers are polyacrylic polymers (sold under the trade name of CARBOPOL™ from B.F. Goodrich), and ethylene acrylic acid copolymers (sold under the trade name PRIMACOR™ from The Dow Chemical Company).

The amount of stabilizing agent varies with polymer composition and solids level but a preferred range of stabilizing agent is from 0.5 weight percent to 10 weight percent based on the total weight of the aqueous pour point depressant dispersion composition. More preferably from about 0.5 weight percent to 7 weight percent, and even more preferably from 0.5 weight percent to 5 weight percent based on the total weight of the aqueous pour point depressant dispersion composition.

If a polyethoxylated nonionic surfactant (i.e., an ethoxy-containing nonionic surfactant) is used in the present invention it may be used alone as the dispersing agent (in the amounts disclosed herein above) or in conjunction with one or more dispersing agent as a stabilizing agent. If it is used in combination with one or more dispersing agent, it is preferably used in an amount equal to or greater than 0.1 weight percent, more preferably in an amount equal to or greater than 0.25 weight percent, more preferably in an amount equal to or greater than 0.5 weight percent, more preferably in an amount equal to or greater than 1 weight percent, and more preferably in an amount equal to or greater than 2 weight percent based on the total weight of the aqueous pour point depressant dispersion composition. If an ethoxy-containing nonionic surfactant is used in combination with one or more dispersing agent in the present invention, it is preferably used in an amount equal to or less than 10 weight percent, more preferably in an amont equal to or less than 7 weight percent, and more preferably in an amount equal to or less than 5 weight percent based on the total weight of the aqueous pour point depressant dispersion composition.

The pour point depressant dispersion compositions of the present invention may contain one or more additional additive or mixtures of additives typically found in such compositions, for example, biocides, colorants, anti-foaming agents, and the like. Such additives are typically added in amounts less than 1 percent by weight based on the total weight of the composition.

The pour point depressant of the present invention is supplied as a dispersion in a liquid medium, preferably comprising water, in which it is not normally soluble at 10°C, and preferably also not soluble at ambient temperature, i.e., about 20°C, or even 30°C or 40°C. That is, the medium is, first, a liquid at ambient temperature (about 20°C) and preferably has a freezing point of 10°C or below. Some preferred media, in particular, mixtures, have freezing points as low as 0°C, -20°C, -30°C, -40°C or below. Moreover, the medium does not dissolve a substantial amount of the pour point depressant at such temperatures, preferably, ambient temperature. More specifically, the medium preferably dissolves less than 4 weight percent, more preferably less than 2 or even 1 weight percent, of the pour point depressant at ambient temperature or moderately elevated temperatures. (In some cases the small soluble fraction may comprise impurities and unreacted materials, so that the amount of actual pour point depressant which dissolves is proportionately even less, e.g., less than 0.5 weight percent.) Preferably the medium remains a non-solvent to 30°C or more preferably to 40°C or 50°C or higher.

In order for the liquid medium to be a nonsolvent for the pour point depressant, the medium should generally have a suitable degree of polarity. Polarity can be measured or expressed in a variety of ways. Thus in one embodiment the molecules of the solvent will preferably have 10 to 80 percent by weight heteroatoms such as oxygen or nitrogen, more preferably 20 to 70 percent, and still more preferably 25 to 60 percent by weight. Alternatively, the medium may have a dielectric constant of at least 3, preferably at least 10. The aforementioned parameters would normally be those of the medium as a whole, including, if it is a mixture, all the components as mixed.

Suitable liquid media include acetates (e.g., 2-ethoxyethyl acetate), ketones (e.g., acetone, butanone, pentanone, hexanone), or preferably, aqueous glycol mixtures (e.g., mixtures of ethylene glycol and water). Among the materials which can be used alone or in combination with water are ethylene glycol and its derivatives, such as the monomethyl ether, the monoethyl ether, the monopropyl ether, the monobutyl ether, and the monohexyl ether; diethylene glycol and its derivatives, such as the monomethyl ether, the monoethyl ether, the monopropyl ether, the monobutyl ether, and the monohexyl ether; propylene glycol and its derivatives, including the monomethyl ether, the monopropyl ether, and the monobutyl ether; and dipropylene glycol and its derivatives, such as the monomethyl ether, the monopropyl ether, and the monobutyl ether.

Other suitable types of materials useful as the liquid medium for the present invention include lactones such as butyrolactone, and alcohols such as butanol, diacetone alcohol (4-hydroxy-4-methyl-2-pentanone) 2,6-dimethyl-4-heptanol, hexanol, isopropanol, 2-ethylhexanol, and 1-pentanol.

The most preferred liquid medium is water. As defined herein, aqueous means containing, dissolved in, or dispersed in water.

Preferably, the aqueous pour point despressant dispersion compositions of the present invention do not conatin any acyclic, cyclic, saturated, unsaturated alkane, arene, or alkylarene hydrocarbon solvents. For example pentane, pentene, hexane, hexene, petroleum ethers, cyclohexane, benzene, toluene, xylenes, gasoline, kerosene, diesel, heavy aromatic naphtha, and the like are not suitable as the liquid medium for the present invention.

Preferably, the aqueous pour point depressant dispersion compositions of the present invention do not contain any such hydrocarbon solvent, in other words, the aqueous pour point depressant dispersion compositions of the present invention are hydrocarbon solvent-free.

In the pour point dispersion compositons of the present invention, the EVA is dispersed not dissolved, in the liquid medium as compared to a solution where the EVA is dissolved in the liquid medium (for example where a hydrocarbon solvent is used).

For many applications and/or environmental conditions, it is advantageous to have an aqueous pour point depressant dispersion with a freezing point equal to or less than 0°C, preferably equal to or less than -10°C, more preferably equal to or less than -20°C, more preferably equal to or less than -30°C, more preferably equal to or less than -40°C, and even more preferably equal to or less than -50°C. It is well known that the freezing point of a solution or mixture is lower than that of the pure solvent, and the degree by which the freezing point is lowered is directly proportional to the molal concentration of the solute. For the present invention, the solution is preferably aqueous, in other words the solvent or primary dispersing liquid medium is water, and the solutes are referred to as freezing point depressants. Different types of solutes useful as freezing point depressants for water are well known in the art and consist of electrolytes such as sodium chloride, potassium chloride, calcium chloride and the like; monohydric alcohols such as methanol, ethanol, propanol, and the like; polyhydric alcohols such as ethylene glycol, propylene glycol and glycerine and the like; glycol ethers such as ethyl, propyl, butyl and hexyl ethers of ethylene glycol; diethylene glycol; propylene glycol and the like. The most preferred are methanol, ethanol, ethylene glycol and propylene glycol since these have the lowest molecular weights, and are relatively inexpensive and readily available. Of these, ethylene glycol is the most preferred for reasons including its non-flammability, low vapor pressure and relatively low environmental impact.

If present, the amount of a freezing point depressant agent incorporated in the aqueous dispersion compositon of the present invention is dictated by the desired freezing point of the aqueous pour point depressant dispersion compositon. In general, one or more such freezing point depressant agent can be used in an amount equal to or greater than 5 weight percent, preferably equal to or greater than 10 weight percent, and more preferably equal to or greater than 15 weight percent based on the final weight of the aqueous pour point depressant dispersion composition. In general, one or more such freezing point depressant agent can be used in an amount equal to or less than 40 weight percent preferably equal to or less than 35 weight percent, and more preferably equal to or less than 30 weight percent based on the final weight of the aqueous pour point depressant dispersion composition.

The liquid medium can also be a mixture of any of the foregoing materials, including mixtures with water, as long as the pour point depressant is substantially insoluble in such mixtures. If the liquid medium is a mixture of a glycol and water, the relative amounts of the materials are such that the water component will not freeze even at low temperatures such as 0°C to -40°C. Preferred weight ratios for such water:glycol mixtures are: 40:60, 50:50, 60:40 to 70:30. However, it is understood that the ratios used are are not held to the ones listed herein above, but are dictated by the ultimate freezing point to be achieved and may be easily determined by one skilled in the art without undue experimentation.

Preferably the liquid medium is used in the present invention in an amount equal to or greater than 35 weight percent, more preferably in an amount equal to or greater than 40 weight percent, and more preferably in an amount equal to or greater than 45 weight percent based on the total weight of the aqueous pour point depressant dispersion composition. Preferably the liquid medium is used in the present invention in an amount equal to or less than 75 weight percent, more preferably in an amont equal to or less than 70 weight percent, and more preferably in an amount equal to or less than 65 weight percent based on the total weight of the aqueous pour point depressant dispersion composition.

While any method may be used, one convenient way to prepare the aqueous pour point dispersion compositions described herein is by melt-kneading. Any melt-kneading means known in the art may be used. In some embodiments a kneader, a Banbury mixer, single-screw extruder, or a multi-screw extruder is used. The melt-kneading may be conducted under the conditions which are typically used for melt-kneading the thermoplastic resin (i). A process for producing the dispersions in accordance with the present invention is not particularly limited. One preferred process, for example, is a process comprising melt-kneading the thermoplastic polymer (i), dispersing agent (ii), and any other additives according to USP 5,756,659; 7,763,676; and 7,935,755, all of which are encorporated herein by reference in their entirety. A preferred melt-kneading machine is, for example, a multi screw extruder having two or more screws, to which a kneading block can be added at any position of the screws. If desired, it is allowable that the extruder is provided with a first material-supplying inlet and a second material-supplying inlet, and further third and forth material-supplying inlets in this order from the upper stream to the down stream along the flow direction of a material to be kneaded. Further, if desired, a vacuum vent may be added at an optional position of the extruder. In some embodiments, the pour point dispersion comprising the thermoplastic polymer, dispersing agent, and any other additives is first diluted to contain about 1 to about 3 percent by weight of water and then subsequently further diluted to comprise greater than 25 percent by weight of water. In some embodiments, the further dilution provides a dispersion with at least about 30 percent by weight of water. The aqueous dispersion obtained by the melt kneading may be further supplemented with a glycol, preferably ethylene glycol. The aqueous pour point depressant dispersions described hereinabove may be used as prepared or diluted further with additional water and/or glycol.

**FIG. 1** schematically illustrates an extrusion apparatus which can be used in the process of the present invention. An extruder **20,** preferably a twin screw extruder, is coupled to a back pressure regulator, melt pump, or gear pump, **30.** Preferably, the apparatus further comprsises a base reservoir **40** and an initial water reservoir **50,** each of which includes a pump (not shown). Desired amounts of base and initial water are provided from the base reservoir **40** and the initial water reservoir **50,** respectively. Any suitable pump may be used, but in some embodiments a pump that provides a flow of about 150 cc/min at a pressure of 240 bar may be used to provide the base and the initial water to the extruder **20.** In other embodiments, a liquid injection pump provides a flow of 300 cc/min at 200 bar or 600 cc/min at 133 bar. In some embodiments the base and initial water are preheated in a preheater.

Thermoplastic EVA polymer, in the form of pellets, powder, or flakes, is fed from the feeder **80** to an inlet **90** of the extruder **20** where the resin is melted or compounded. In some embodiments, the dispersing agent and/or stabilizing agent is added to the resin through an opening along with the resin and in other embodiments, the dispersing agent and/or stabilizing agent is provided separately to the twin screw extruder **20.** The resin melt is then delivered from the mix and convey zone to an emulsification zone of the extruder where the initial amount of water and base from the reservoirs **40** and **50** is added through inlet **55.** In some embodiments, dispersing agent may be added additionally or exclusively to the water stream. In one embodiment wherein the dispersing agent is a salt of a fatty acid, the dispersing agent may be added as the salt of the fatty acid, or it may be added to the extruder as the fatty acid which is converted to its salt form in the extruder. In some embodiments, the emulsified mixture is further diluted with additional water and/or glycol and/or stabilizing agent via inlet **95** from reservoir **60** in a dilution and cooling zone of the extruder **20.** Typically, the dispersion is diluted to at least 30 weight percent water in the cooling zone. In addition, the diluted mixture may be diluted any number of times until the desired dilution level is achieved.

In one method to make the aqueous pour point despressant dispersions of the present invention, step a, the combination of ethylene vinyl acetate (EVA), a dispersing agent; and water to form an aqueous dispersion of EVA, and step b mixing the aqueous dispersion of EVA with iv an aqueous freezing point depressant to form the aqueous pour point depressant dispersion composition, are conducted sequentially in the extruder in which the aqueous dispersion of EVA is produced.

In a prefered method to make the aqueous pour point despressant dispersions of the present invention, some or all of the water and/or glycol and/or stabilizing agent is not added into the twin screw extruder **20** but rather to a stream containing the dispersed polymer after it has exited from the extruder. In other words, step b does not occur in the extruder in which the aqueous dispersion of EVA is produced. In this manner, steam pressure build-up in the extruder **20** is minimized.

In some embodiments a basic substance or aqueous solution, dispersion or slurry thereof is added to the dispersion at any point of the process, preferably to the extruder. Typically the basic substance is added as an aqueous solution. But in some embodiments, it is added in other convenient forms, such as pellets or granules. In some embodiments, the basic substance and water are added through separate inlets of the extruder. Examples of the basic substance which may be used for the neutralization or the saponification in the melt kneading process include alkaline metals and alkaline earth metals such as sodium, potassium, calcium, strontium, barium; inorganic amines such as hydroxylamine or hydrazine; organic amines such as methylamine, ethylamine, ethanolamine, cyclohexylamine, tetramethylammonium hydroxide; oxide, hydroxide, and hydride of alkaline metals and alkaline earth metals such as sodium oxide, sodium peroxide, potassium oxide, potassium peroxide, calcium oxide, strontium oxide, barium oxide, sodium hydroxide, potassium hydroxide, calcium hydroxide, strontium hydroxide, barium hydroxide, sodium hydride, potassium hydride, calcium hydride; and weak acid salts of alkaline metals and alkaline earth metals such as sodium carbonate, potassium carbonate, sodium hydrogencarbonate, potassium hydrogencarbonate, calcium hydrogencarbonate, sodium acetate, potassium acetate, calcium acetate; or ammonium hydroxide. In particular embodiments, the basic substance is a hydroxide of an alkaline metal or a hydroxide of an alkali metal. In some embodiments, the basic substance is selected from potassium hydroxide, sodium hydroxide and combinations thereof.

The thermoplastic polymer of the aqueous pour point depressant dispersion compositions of the present invention has an advantageous particle size distribution. In particular embodiments, the dispersed thermoplastic polymer has a particle size distribution defined as volume average particle diameter (Dv) divided by number average particle diameter (Dn) of equal to or less than 2.5, preferably equal to or less than 2.0. In other embodiments, the dispersions have a particle size distribution of less than or equal to 1.9, 1.7, or 1.5.

A preferred volume average particle size is equal to or less then 2 micron (µm), preferably equal to or less than 1.5 µm, preferably equal to or less than 1.2 µm, and more preferably equal to or less than 1 µm. In other embodiments, the average particle size ranges from 0.05 µm to 1 µm. In still other embodiments, the average particle size of the dispersion ranges from 0.5 µm to 1.2 µm, preferably 0.5 µm to 1 µm. For particles that are not spherical the diameter of the particle is the average of the long and short axes of the particle. Particle sizes can be measured on a Coulter LS230 light-scattering particle size analyzer or other suitable device.

The dispersions of the present invention have a pH of from about 5 to about 13.5, preferably from about 8 to about 13, more preferably from about 10 to about 12.

In a preferred embodiment, this invention is a method of inhibiting the deposition of paraffins (also referred to as wax) and/or improving the flow properties of oil comprising adding to the oil an effective amount of the pour point depressant dispersion composition of this invention. Effective EVA polymer doses are typically from 1 part per million (ppm) to 5,000 ppm, preferably 10 ppm to 300 ppm.

The pour point depressant dispersion composition of the present invention can be added to an oil pipeline by batch or continuous injection or squeezing, upstream or downstream of the location of any potential cold area likely to result in deposition of wax, gellation, thickening, sludging, etc. Also, the polymer composition can be added at the cold area (reservoir, tank, container, etc.) to decrease the pour point of the oil. The oil may be crude oil, condensate, middle distillate, fuel oil, diesel, etc.

The pour point dispersant dispersion compositions of the present invention may be used alone or in combination with other additives including dewaxing auxiliaries, corrosion inhibitors, asphaltene inhibitors, scale inhibitors, antioxidants, lubricity additives, dehazers, conductivity improvers, cetane number improvers, sludge inhibitors, and the like.

The foregoing may be better understood by the following Examples, which are presented for purposes of illustration and are not intended to limit the scope of this invention.

### EXAMPLES

### Example 1

One hundred (100) parts by weight of an ethylene vinyl acetate copolymer comprising about 28 weight percent vinyl acetate, having a density of about 0.95 g/cc (as determined according to ASTM D-792), a melt index of about 43 g/10 minutes (ASTM D1238 at 190°C and 2.16 kg), and a DSC melting point of about 74°C (ASTM 3418), commercially available from DuPont as ELVAX™ 240W and 3.0 parts by weight of a fractionated fatty acid, available from Croda Inc. under the tradename PRIFRAC™ 2989 (comprising about 88 percent behenic acid) are melt kneaded at 190°C in a twin screw extruder at a rate of 6.2 kg/hr.

Upon the melt kneaded resin/stabilizing agent, 7.5 weight percent aqueous solution of potassium hydroxide is continuously fed into a downstream injection port at a rate of 0.37 kg/hr. This aqueous dispersion is subsequently diluted with additional water at a rate of 5.6 kg/hr before exiting the extruder.

An aqueous dispersion having a solids content of 51.2 weight percent and a pH of 11.2 is obtained. The dispersed polymer phase measured by a Coulter LS 13 320 particle analyzer consists of an average volume diameter of 0.41 micrometers and a particle size distribution (Dv/Dn) of 1.13.

### Examples 2 to 5

To the dispersion from Example 1 is added a water/ethylene glycol mixture, comprising an optional surfactant, to attain an aqueous pour point depressant dispersion of the present invention. It is not critical to the invention in which order the components are added together. Each composition has 30 percent by weight solids. In Examples 2 to 5 the following order of addition is used. The (optional) surfactant is added to the ethylene glycol, the (optional)surfactant/ethylene glycol mixture is then added to the dispersion of Example 1 to give a final ratio of water to ethylene glycol of 50:50 by weight. This offers protection against freezing to about -40°C. Mixing is accomplished using a magnetic stirrer, but any any general technique may be used, including (but not limited to) magnetic stirring, mechanical mixing such as a blender, overhead mixing equipment, and the like. The compositions of Examples 2 to 5 are described in Table 1, amounts are listed as weight percent based on the total weight of the aqueous pour point depressant dispersion compositon. About 10 grams of the composition is placed into 20ml screw thread vials (Fisher Scientific #03-339-5A) and placed into a dry ice/acetone bath at -40°C to equilibrate. After equilibration, the vial is removed from the bath and tipped slightly. If the composition moves when the vial is tipped it is classified as flowing at -40°C. Grit is a term that is used to describe larger particles that form when the ethylene glycol is added to the dispersion, or when the dispersion is added to the ethylene glycol. It is believed that these large particles can form from the destabilization of the small particles which agglomerate and form large particles that can easily be seen without any magnification required.

**Table 1**

| Example | Surfactant | wt % | Water:EG | Solids, wt % | Grit | Flow @ -40°C |
|---|---|---|---|---|---|---|
| 2 | None | | 50:50 | 30 | yes | yes |
| 3 | NEODOL 91-2.5 | 0.5 | 50:50 | 30 | no | yes |
| 4 | ECOSURF EH-3 | 0.5 | 50:50 | 30 | no | yes |
| 5 | TERGITOL NP-4 | 0.5 | 50:50 | 30 | no | yes |

### Example 6

Ninety seven (97) parts by weight of an ethylene vinyl acetate copolymer comprising about 32 weight percent vinyl acetate, a density of about 0.96 g/cc (as determined according to ASTM D-792), a melt index of about 43 g/10 minutes (ASTM D1238 at 190°C and 2.16 kg), and a DSC melting point of about 63°C (ASTM 3418), commercially available from DuPont as ELVAX 150, and 3.0 parts by weight of a fractionated fatty acid, available from Croda Inc. under the tradename PRIFRAC™ 2989 (comprising about 88 percent behenic acid) are melt kneaded at 150°C in a twin screw extruder at a rate of 6.0 kg/hr.

Upon the melt kneaded resin/stabilizing agent, 10.7 weight percent aqueous solution of potassium hydroxide is continuously fed into a downstream injection port at a rate of 0.31 kg/hr. This aqueous dispersion is subsequently diluted with additional water at a rate of 5.8 kg/hr before exiting the extruder.

An aqueous dispersion having a solids content of 50.9 weight percent and a pH of 11.6 is obtained. The dispersed polymer phase measured by a Coulter LS 13 320 particle analyzer consisted of an average volume diameter of 0.42 micrometers and a particle size distribution (Dv/Dn) of 1.15.

### Examples 7 to 10

Examples 7 to 10 are prepared as described herein above for Examples 2 to 5 except that Example 6 is the dispersion added to a water/glycol mixture comprising an optional surfactant to attain an aqueous pour point depressant dispersion of the present invention. The compositions and performance for Examples 7 to 10 are described in Table 2, amounts are listed as weight percent based on the total weight of the aqueous pour point depressant dispersion compositon.

**Table 2**

| Example | Surfactant | wt % | Water:EG | Solids, wt % | Grit | Flow @ -40°C |
|---|---|---|---|---|---|---|
| 7 | None | | 50:50 | 30 | yes | yes |
| 8 | NEODOL 91-2.5 | 3 | 50:50 | 30 | no | yes |
| 9 | ECOSURF EH-3 | 3 | 50:50 | 30 | no | yes |
| 10 | TERGITOL NP-4 | 3 | 50:50 | 30 | no | yes |

Pour point depression for Examples 2 to 5 and 7 to 10 is demonstrated by measuring the reduction in pour point of a wax-containing crude oil (Comparative Example A). For the test, a waxy crude oil with API gravity of 30 (density of 0.877 g/ml) and a pour point (untreated) of 39°C is treated with a pour point depressant of the present invention at a treat rate of 750 parts per million (ppm) or 1500 ppm.

The crude oil is first beneficiated by holding it in a tightly sealed container at about 60°C for at least 3 hours to erase thermal history and homogenize the crude oil mixture. Using a repeat pipetter with disposable tips, the hot oil is dispensed into a sample vial containing the appropriate amount of pour point depressant according to the desired treat rate. The vial is then capped tightly and held at about 60°C for at least two hours with intermittent shaking in order to allow the pour point depressant to interact with the crude oil. The pour point of the mixture is measured using an automated MPP 5Gs instrument available from Instrumentation Scientifique de Laboratoire. The pour point measurements obtained with this instrument are correlated with ASTM D-97 "Standard Test Method for Pour Point of Petroleum Products". Replicate measurements are performed.

The results are shown in Table 3. Comparative Example A is the crude oil without additive, Comparative Example B is the crude oil of Comparative Example A treated with a 10% solution of EVA in toluene (the same EVA used in Example 1), and Comparative Example C is the crude oil of Comparative Example A treated with a 10% solution of EVA in toluene (the same EVA used in Example 6). The values reported are accurate within about ±3 °C. As can be seen from the values reported in Table 3, the pour point despression compositions of the preent invention can reduce the pour point by 9°C or more. Further, it can be seen that the results are equal or very comparable to those obtained using a toluene solution of the same polymer.

**Table 3**

| Example | Pour point, °C @ 750 ppm | Pour point, °C @ 1500 ppm |
|---|---|---|
| A | 39* | 39* |
| B | 31 | 23 |
| 2 | 30 | 30 |
| 3 | 32 | 27 |
| 4 | 32 | 27 |
| 5 | 32 | 28 |
| C | 35 | 28 |
| 7 | 35 | 28 |
| 8 | 35 | 28 |
| 9 | 35 | 28 |
| 10 | 35 | 28 |

| | | |
|---|---|---|
| *No pour point depressant added. | | |

## Claims

1. A method to make an aqueous pour point depressant dispersion composition comprising the steps of:
a combining in an extruder
i ethylene vinyl acetate copolymer (EVA),
ii a dispersing agent;
and
iii water
to form an aqueous dispersion of EVA,
and
b mixing the aqueous dispersion of EVA with
iv an aqueous freezing point depressant to form the aqueous pour point depressant dispersion composition,
wherein the amount of EVA in the final composition is from 25 to 60 weight percent, wherein the average particle size of the dispersed EVA is equal to or less than 1 micrometer, the amount of dispersing agent in the final composition is from 1 to 10 weight percent, the water: aqueous freezing point depressant mixture in the final composition is present in an amount of from 40 to 75 weight percent, the water: aqueous freezing point depressant ratio is from 40:60 to 70:30, and weight percents are based on the total weight of the aqueous pour point depressant dispersion composition.

2. The method of Claim 1 wherein the pour point depressant composition further comprises:
V a stabilizing agent in an amount of from 0.5 weight percent to 5 weight percent.

3. The method of Claim 1 wherein the dispersing agent ii comprises one or more of:
a a fatty acid/salt having the formula R₁COOR₂ wherein R₁ is a straight chain, saturated or unsaturated, hydrocarbon radical of 8 to 25 carbon atoms and R₂ is H or a base-forming radical;
b an alkyl, arene and/or alkylarene sulfonate;
c a salt of a polymer of alkyl acrylate and/or alkyl methacrylate and acrylic and/or methacrylic acid, or a salt of partial esters of maleic anhydride-styrene copolymers;
d a cationic surfactant;
e a zwitterionic surfactant;
or
f a nonionic surfactant.

4. The method of Claim 1 wherein the dispersing agent ii is a sodium salt of oleic acid, stearic acid, behenic acid or erucic acid or a potassium salt of oleic acid, stearic acid, behenic acid or erucic acid, or mixtures thereof.

5. The method of Claim 1 wherein the stabilizing agent V is a polyethoxylated nonionic surfactant and the aqueous freezing point depressant is ethylene glycol.

6. The method of Claim 1 wherein the pour point depressant dispersion composition further comprises one or more additional additive vi selected from a biocide, a colorant, or an anti-foaming agent.

7. The method of Claim 1 wherein steps a and b are conducted sequentially in the extruder in which the aqueous dispersion of EVA is produced.

8. The method of Claim 1 wherein step b does not occur in the extruder in which the aqueous dispersion of EVA is produced.

9. The method of Claims 3 and 4 wherein the salt is formed in the extruder.

## Patentansprüche

1. Ein Verfahren zum Herstellen einer wässrigen Pourpointerniedriger-Dispersionszusammensetzung, das die folgenden Schritte beinhaltet:
a Kombinieren in einem Extruder von Folgendem:
i Ethylenvinylacetatcopolymer (EVA),
ii einem Dispergiermittel;
und
iii Wasser
zum Bilden einer wässrigen EVA-Dispersion
und
b Mischen der wässrigen EVA-Dispersion mit
iv einem wässrigen Erstarrungspunkterniedriger zum Bilden der wässrigen Pourpointerniedriger-Dispersionszusammensetzung,
wobei die EVA-Menge in der Endzusammensetzung 25 bis 60 Gewichtsprozent beträgt, wobei die durchschnittliche Partikelgröße des dispergierten EVA gleich oder kleiner als 1 Mikrometer ist, die Menge an Dispergiermittel in der Endzusammensetzung 1 bis 10 Gewichtsprozent beträgt, die Mischung von Wasser und wässrigem Erstarrungspunkterniedriger in der Endzusammensetzung in einer Menge von 40 bis 75 Gewichtsprozent vorhanden ist, das Verhältnis von Wasser zu dem wässrigen Erstarrungspunkterniedriger 40 : 60 bis 70 : 30 beträgt und sich die Gewichtsprozente auf das Gesamtgewicht der wässrigen Pourpointerniedriger-Dispersionszusammensetzung beziehen.

2. Verfahren gemäß Anspruch 1, wobei die Pourpointerniedriger-Zusammensetzung ferner Folgendes beinhaltet:
V ein Stabilisierungsmittel in einer Menge von 0,5 Gewichtsprozent bis 5 Gewichtsprozent.

3. Verfahren gemäß Anspruch 1, wobei das Dispergiermittel ii eines oder mehrere von Folgendem beinhaltet:
a einer Fettsäure/einem Salz mit der Formel R₁COOR₂, wobei R₁ ein geradkettiger, gesättigter oder ungesättigter Kohlenwasserstoffrest aus 8 bis 25 Kohlenstoffatomen ist und R₂ H oder ein Basen bildender Rest ist;
b einem Alkyl-, Aren- und/oder Alkylarensulfonat;
c einem Salz eines Polymers von Alkylacrylat und/oder Alkylmethacrylat und Acryl- und/oder Methacrylsäure oder einem Salz von Teilestern von Maleinsäureanhydrid-Styrol-Copolymeren;
d einem kationischen Tensid;
e einem zwitterionischen Tensid;
oder
f einem nichtionischen Tensid.

4. Verfahren gemäß Anspruch 1, wobei das Dispergiermittel ii ein Natriumsalz von Ölsäure, Stearinsäure, Behensäure oder Erucasäure oder ein Kaliumsalz von Ölsäure, Stearinsäure, Behensäure oder Erucasäure oder Mischungen davon ist.

5. Verfahren gemäß Anspruch 1, wobei das Stabilisierungsmittel V ein polyethoxyliertes nichtionisches Tensid ist und der wässrige Erstarrungspunkterniedriger Ethylenglycol ist.

6. Verfahren gemäß Anspruch 1, wobei die Pourpointerniedriger-Dispersionszusammensetzung ferner ein oder mehrere zusätzliche Zusatzstoffe vi, ausgewählt aus einem Biozid, einem Farbmittel oder einem Antischaummittel, beinhaltet.

7. Verfahren gemäß Anspruch 1, wobei die Schritte a und b sequenziell in dem Extruder ausgeführt werden, in dem die wässrige EVA-Dispersion produziert wird.

8. Verfahren gemäß Anspruch 1, wobei Schritt b nicht in dem Extruder stattfindet, in dem die wässrige EVA-Dispersion produziert wird.

9. Verfahren gemäß den Ansprüchen 3 und 4, wobei das Salz in dem Extruder gebildet wird.

## Revendications

1. Une méthode de préparation d'une composition de dispersion aqueuse abaissant le point d'écoulement, comprenant les étapes de :
a combinaison, dans une extrudeuse,
i d'un copolymère acétate de vinyle-éthylène (EVA),
ii d'un agent dispersant ;
et
iii d'eau
pour former une dispersion aqueuse du copolymère EVA
et
b mélange de la dispersion aqueuse du copolymère EVA avec
iv un abaisseur aqueux du point de congélation pour former la composition de dispersion aqueuse abaissant le point d'écoulement,
la quantité de copolymère EVA dans la composition finale allant de 25 à 60 pour cent en poids, la dimension moyenne des particules du copolymère EVA dispersé étant inférieure ou égale à 1 micromètre, la quantité d'agent dispersant dans la composition finale allant de 1 à 10 pour cent en poids, le mélange eau/abaisseur aqueux du point de congélation étant présent dans la composition finale à une quantité allant de 40 à 75 pour cent en poids, le rapport eau/abaisseur aqueux du point de congélation allant de 40/60 à 70/30 et les pourcentages en poids étant rapportés au poids total de la composition de dispersion aqueuse abaissant le point d'écoulement.

2. La méthode de la revendication 1 où la composition abaissant le point d'écoulement comprend en outre :
V un agent stabilisant à une quantité allant de 0,5 pour cent en poids à 5 pour cent en poids.

3. La méthode de la revendication 1 où l'agent dispersant ii comprend un ou plusieurs des suivants :
a un acide gras/sel de formule R₁COOR₂, R₁ étant un radical hydrocarbure saturé ou insaturé à chaîne linéaire à 8 à 25 atomes de carbone et R₂ étant un H ou un radical formant une base ;
b un sulfonate d'alkyle, d'arène et/ou d'alkylarène ;
c un sel d'un polymère d'acrylate d'alkyle et/ou de méthacrylate d'alkyle et d'un acide acrylique et/ou méthacrylique ou un sel d'esters partiels de copolymères anhydride maléique-styrène ;
d un agent tensioactif cationique ;
e un agent tensioactif zwitterionique ;
ou
f un agent tensioactif non ionique.

4. La méthode de la revendication 1 où l'agent dispersant ii est un sel de sodium d'un acide oléique, d'un acide stéarique, d'un acide béhénique ou d'un acide érucique ou un sel de potassium d'un acide oléique, d'un acide stéarique, d'un acide béhénique ou d'un acide érucique ou des mélanges de ceux-ci.

5. La méthode de la revendication 1 où l'agent stabilisant V est un agent tensioactif non ionique polyéthoxylé et l'abaisseur aqueux du point de congélation est l'éthylène glycol.

6. La méthode de la revendication 1 où la composition de dispersion abaissant le point d'écoulement comprend en outre un ou plusieurs additifs supplémentaires vi sélectionnés parmi un biocide, un colorant ou un agent antimousse.

7. La méthode de la revendication 1 où les étapes a et b sont effectuées séquentiellement dans l'extrudeuse dans laquelle la dispersion aqueuse du copolymère EVA est produite.

8. La méthode de la revendication 1 où l'étape b n'a pas lieu dans l'extrudeuse dans laquelle la dispersion aqueuse du copolymère EVA est produite.

9. La méthode des revendications 3 et 4 dans laquelle le sel est formé dans l'extrudeuse.
